# FASCICULE DE BREVET EUROPEEN

(11) **EP 1 408 917 B1**
(45) Date de publication et mention de la délivrance du brevet: **20.09.2006**
(21) Numéro de dépôt: 02764955.7
(22) Date de dépôt: 09.07.2002
(51) Int. Cl.: A61K 8/33, A61Q 3/02, A61Q 3/00

(54) **DURCISSEUR POUR LES ONGLES**
NAGELHÄRTER
NAIL HARDENER

(30) Priorité: 10.07.2001 FR 0109127
(43) Date de publication de la demande: 21.04.2004
(73) Titulaire: Fiabila, 28130 Maintenon (FR)
(72) Inventeur: RENARD, Christine, F-28130 Maintenon (FR)
(74) Mandataire: Laget, Jean-Loup
(86) Numéro de dépôt international: PCT/FR2002/002399
(87) Numéro de publication internationale: WO 2003/005975

(56) Documents cités:
- US-A- 4 996 043
- US-A- 5 190 979
- US-A- 5 484 597
- OPDYKE D.L.J. FD. COSMET. TOXICOL. vol. 17, 1979, pages 241 - 275, XP000617419
- DATABASE WPI Week 199539, Derwent Publications Ltd., London, GB; Class B04, AN 1995-300910 & RU 2 029 544 C1 (GUSEV V V) 27 Février 1995

## Description

La présente invention concerne un composé durcisseur pour les ongles, et son utilisation dans des compositions cosmétiques pour les ongles, notamment les vernis à ongles.

Dans le domaine des vernis à ongles, le formol a jusqu'à présent été largement utilisé dans des proportions de l'ordre de 3 à 4 % en poids pour durcir les ongles.

Cependant, cette molécule est de plus en plus fréquemment classée dans les produits susceptibles de provoquer des allergies, c'est pourquoi on cherche maintenant à limiter son usage, voire à le remplacer totalement.

Dans ses recherches d'actifs pour durcir les ongles, équivalents au formol, la demanderesse a découvert que le citral (3,7-diméthyl 2,6-octanedial) présente la propriété de durcir les ongles. Le citral d'origine naturelle en particulier, mélange de deux isomères le géranial et le néral, possède cette propriété régénératrice de la structure des ongles.

Cet aldéhyde réagit comme le formol pour améliorer la structure de l'ongle, sans en avoir la toxicité.

On extrait ce citral naturel des huiles essentielles de certaines plantes, parmi lesquelles :
- Backhousia citriodora qui contient environ 95-97 % en poids de citral
- Litsea citrata " 90 % "
- Cymbopogon flexuosus " 75 % "
- Feuilles de Leptospermum liversidgei " 70-80 % "
- Litsea cubeba " 70 % "
- Ocimum grotissimum " 66,5% "
- Lindera citriodora " 65 % "
- Calypranthes parriculata " 62% "
- Feuille de Citrus aurantifolia " 36% "
- Citron vert " 6-9% "
- Citron " 2-5% "

Les informations ci-dessus sont tirées de Fenaroli's Handbook of Flavor Ingredients, 1975, cité par Food Cosmet. Toxicol, vol 17, p. 259-266, 1979.

Jusqu'à maintenant, le citral était utilisé principalement dans le domaine alimentaire comme agent de goût et dans le domaine cosmétique (savons, crèmes, bases parfumantes) en tant que parfum, à des concentrations variant approximativement entre 0,002 et 0,8 % en poids. (voir Tableau I).

**Tableau I : Teneur en citral de diverses compositions (% en poids) (Food Cosmet. Toxicol., vol 17, p. 259-266, 1979)**

| Concentration | Savons | Produits de lavage | Crèmes, lotions | Parfums |
|---|---|---|---|---|
| Classique | 0,02 | 0,002 | 0,005 | 0,2 |
| Maximale | 0,2 | 0,02 | 0,02 | 0,8 |

La présente invention concerne donc une nouvelle utilisation du citral, à savoir en tant que durcisseur des ongles. De préférence ce citral est d'origine naturelle, extrait de plantes, l'extrait renfermant au moins 2 % en poids de citral, avantageusement au moins 60 %.

De manière préférée, le citral est donc utilisé dans une composition cosmétique pour les ongles, pouvant renfermer jusqu'à 5 % en poids de citral, avantageusement jusqu'à 0,1 % en poids, ou même seulement jusqu'à 0,05 % en poids.

De manière tout à fait avantageuse, le citral est utilisé dans une composition cosmétique pour les ongles ne renfermant aucun autre aldéhyde, et principalement ne renfermant pas de formol. Ceci permet notamment d'éviter tous les problèmes toxicologiques ou d'allergies inhérents à l'utilisation du formol.

Dans les vernis à ongles, les différents tests ont montré qu'une faible concentration, à savoir entre 0,001 et 0,05 % environ, peut apporter une nette amélioration de l'aspect physique des ongles. L'effet du citral se traduit par des ongles moins fragiles, moins cassants, présentant moins de dédoublements, moins de fissures, plus épais avec une surface plus régulière. Cette amélioration augmente également avec la concentration en citral.

La présente invention concerne notamment l'utilisation du citral dans un vernis à ongles coloré ou incolore, dans une sous-couche de vernis, ou encore dans une base de soins pour les ongles.

L'invention est illustrée par les exemples suivants :

### EXEMPLES :

### Exemple 1 :

Utilisation du citral en tant que durcisseur dans un vernis coloré.

Dans ce vernis, le citral utilisé est une huile essentielle obtenue à partir de la "Verveine des Indes" (Cymbopogon citratus).

Le vernis présente la composition suivante (% en poids) :
- Acétate de butyle 32
- Acétate d'éthyle 28,27
- Nitrocellulose 12
- Résine polyester 7
- Alcool isopropylique 5,2
- Acétyltributylcitrate 5
- Résine tosylamide/époxy 4
- Stéaralkonium hectorite 1,1
- Résine styrène/acrylique 1
- Benzophénone-1 0,1
- Diméthicone 0,01
- Citral 0,02
- Mica 2
- DC Red 7 1,2
- DC Red 34 0,8
- Dioxyde de titane 0,3

Cette composition a été testée sur un échantillon de 21 personnes pendant un mois, dans des conditions normales d'emploi (vernis enlevé et réappliqué tous les 2 à 4 jours).

Les résultats des observations des utilisateurs sont regroupés dans le tableau II ci-après:

**Tableau II : Nombre de sujets ayant constaté une amélioration (nombre de sujets testés : 21)**

| Amélioration | Légère | Modérée | Nette | Très nette | Total |
|---|---|---|---|---|---|
| Fragilité des ongles | 4 | 3 | 3 | 4 | 14 (67 %) |
| Ongles cassants | 7 | 2 | 1 | 5 | 15(71%) |
| Ongles fissurés | 5 | 1 | 4 | 4 | 14 (67 %) |
| Ongles dédoublés | 7 | 4 | 2 | 3 | 16 (76 %) |
| Ongles fins | 8 | 2 | 2 | 4 | 16 (76 %) |
| Dureté des ongles | 5 | 4 | 3 | 5 | 17 (80 %) |
| Surface de l'ongle | 3 | 1 | / | 2 | 6 (29 %) |

Plus des deux tiers des sujets ont remarqué une amélioration de l'aspect de leurs ongles en ce qui concerne la fragilité et dureté des ongles, les ongles cassants, les ongles fissurés, les ongles dédoublés, les ongles fins. En outre, 29 % des sujets ont noté une amélioration du relief de la surface de l'ongle.

Ces résultats prouvent que le citral possède des propriétés de durcissement des ongles même à une teneur aussi faible que 0,02 % en poids dans un vernis.

### Exemple 2 :

Dans ce vernis incolore le citral utilisé est obtenu par distillation de l'huile du Litsea Cubeba. Cette fraction contient 92 % de citral.
- Acétate de butyle 35
- Acétate d'éthyle 33,85
- Acétobutyrate de cellulose 7
- Nitrocellulose 5
- Résine polyester 8
- Acétyltributylcitrate 6
- Résine tosylamide/époxy 5
- Benzophénone-1 0,1
- Diméthicone 0,01
- Citral 0,04

Des tests effectués dans des conditions identiques à ceux de l'exemple 1 ont permis de noter que le nombre des satisfaits restait le même, avec une proportion des très satisfaits plus importante.

## Revendications

1. Utilisation du citral en tant que durcisseur pour les ongles.

2. Utilisation selon la revendication 1, **caractérisée en ce que** le citral est d'origine naturelle.

3. Utilisation selon la revendication 2, **caractérisée en ce que** le citral est extrait de plantes, renfermant au moins 2 % en poids de citral.

4. Utilisation selon l'une des revendications 1 à 3 dans une composition cosmétique pour les ongles.

5. Utilisation selon la revendication 4, **caractérisée en ce que** la composition renferme jusqu'à 5 % en poids de citral.

6. Utilisation selon la revendication 5, **caractérisée en ce que** la composition renferme jusqu'à 0,1 % en poids de citral.

7. Utilisation selon la revendication 4 ou 5, **caractérisée en ce que** la composition renferme jusqu'à 0,05 % en poids de citral.

8. Utilisation selon l'une des revendications 4 à 7, **caractérisée en ce que** la composition ne renferme pas de formol.

9. Utilisation selon l'une des revendications 1 à 8 dans un vernis à ongles coloré ou incolore, dans une sous-couche de vernis, ou dans une base de soins pour les ongles.

## Claims

1. The use of citral as a hardener for nails.

2. The use according to Claim 1, **characterised in that** the citral is of natural origin.

3. The use according to Claim 2, **characterised in that** the citral is extracted from plants, containing at least 2% by weight of citral.

4. The use according to one of Claims 1 to 3 in a cosmetic composition for nails.

5. The use according to Claim 4, **characterised in that** the composition contains up to 5% by weight of citral.

6. The use according to Claim 5, **characterised in that** the composition contains up to 0.1 % by weight of citral.

7. The use according to Claim 4 or 5, **characterised in that** the composition contains up to 0.05% by weight of citral.

8. The use according to one of Claims 4 to 7, **characterised in that** the composition does not contain formal.

9. The use according to one of Claims 1 to 8 in a coloured or clear nail varnish, in a varnish undercoat, or in a nail care base.

## Patentansprüche

1. Verwendung von Citral als Nagelhärter.

2. Verwendung nach Anspruch 1, **dadurch gekennzeichnet, daß** das Citral natürlichen Ursprungs ist.

3. Verwendung nach Anspruch 2, **dadurch gekennzeichnet, daß** das Citral aus Pflanzen extrahiert ist, wobei das Extrakt mindestens 2 Gew.-% Citral enthält.

4. Verwendung nach einem der Ansprüche 1 bis 3 in einer kosmetischen Zusammensetzung für die Nägel.

5. Verwendung nach Anspruch 4, **dadurch gekennzeichnet, daß** die Zusammensetzung bis zu 5 Gew.-% Citral enthält.

6. Verwendung nach Anspruch 5, **dadurch gekennzeichnet, daß** die Zusammensetzung bis zu 0,1 Gew.-% Citral enthält.

7. Verwendung nach Anspruch 4 oder 5, **dadurch gekennzeichnet, daß** die Zusammensetzung bis zu 0,05 Gew.-% Citral enthält.

8. Verwendung nach einem der Ansprüche 4 bis 7, **dadurch gekennzeichnet, daß** die Zusammensetzung kein Formol enthält.

9. Verwendung nach einem der Ansprüche 1 bis 8 in einem farbigen oder farblosen Nagellack, in einem Unterlack oder in einer Basispflege für Nägel.
